# EUROPÄISCHE PATENTSCHRIFT

(11) **EP 2 373 366 B1**
(45) Veröffentlichungstag und Bekanntmachung des Hinweises auf die Patenterteilung: **17.02.2016**
(21) Anmeldenummer: 09796651.9
(22) Anmeldetag: 27.11.2009
(51) Int. Cl.: A61M 11/00, A61M 15/00

(54) **INHALATOR**
INHALER
INHALATEUR

(30) Priorität: 03.12.2008 EP 08170606
(43) Veröffentlichungstag der Anmeldung: 12.10.2011
(73) Patentinhaber: Boehringer Ingelheim International GmbH, 55216 Ingelheim am Rhein (DE)
(72) Erfinder: JUNG, Andree, 55216 Ingelheim am Rhein (DE)
(74) Vertreter: Simon, Elke Anna Maria
(86) Internationale Anmeldenummer: PCT/EP2009/065949
(87) Internationale Veröffentlichungsnummer: WO 2010/063645

(56) Entgegenhaltungen:
- EP-A1- 1 844 806
- WO-A1-00/45879
- WO-A1-95/28192
- WO-A1-99/36116
- US-A1- 2007 181 123

## Beschreibung

Die Erfindung bezieht sich auf einen Inhalator, insbesondere Pulverinhalator, zur Verabreichung eines Arzneimittels in Form inhalationsfähiger Substanzen, Substanzformulierungen oder -mischungen mit einem Mundstück und einem Magazin, das ein Gehäuse mit mehreren Kavitäten zur Aufnahme des Arzneimittels umfasst, wobei das Mundstück mit jeweils einer der Kavitäten in strömungsverbindung steht.

Aus dem Stand der Technik sind Inhalationsgeräte bekannt, bei denen durch eine beim Inhalieren erzeugte Unterdruckströmung (Venturi) direkt an eine Medikamentenkammer angeschlossen und ein darin befindliches pulverförmiges Medikament entnommen wird. Bei einem Inhalator nach der US-A-6 655 381 ist in einem Ringmagazin in kreisförmig angeordneten Kavitäten ein pulverförmiges Medikament gelagert. Ein die Kavitäten schließender Siegel wird im Wesentlichen als Ganzes entfernt und ein Venturirohr mit einer Verengung wird über der Kavität parallel zum Magazin angeordnet. An das Venturirohr ist eine längere Verwirbelungskammer angeschlossen, um das Medikament im Wesentlichen auf einmal der Kavität zu entnehmen.

Darüber hinaus existieren Inhalatoren, in denen eine Medikamentenkammer durch Anstechen geöffnet wird. Die dabei entstehenden Öffnungen in einer Siegelfolie sind jedoch nicht sehr genau definiert.

Darüber hinaus beschreibt die EP 1 844 806 A1 einen Mehrdosispulverinhalator, bei dem in einem Luftkanal eine Antriebsströmung erzeugt wird, die aufgrund einer Verengung im Luftkanal in diesem Bereich eine Unterdruckströmung erzeugt. Der die Unterdruckströmung verursachende engste Bereich des Luftkanals ist dabei an eine Entnahmeöffnung einer Medikamentenkammer angeschlossen. Die Entnahmeöffnung steht mit einer Steueröffnung in Verbindung, so dass eine Entleerungsströmung durch die Steueröffnung via Entnahmeöffnung und durch die Medikamentenkammer gebildet wird. Die Entleerungsströmung ist dabei vor Eintritt in die Medikamentenkammer nicht mit der Antriebsströmung verbunden, vereinigt sich jedoch nach Austritt aus der Entnahmeöffnung mit der Antriebsströmung, wodurch ein mit der Entleerungsströmung mitgeführtes Medikament mit der Antriebsströmung in Richtung Mundstück transportiert wird. Das Medikamentenmagazin weist typischerweise zusätzlich zu der Steuer- und Entnahmeöffnung, eine Einfüllöffnung auf, die unabhängig von den beiden anderen Öffnungen im Magazin eingebracht ist und die relativ groß sein kann, um ein Befüllen zu erleichtern. Die Einfüllöffnung wird nach dem Befüllen mit dem Pulver mit einer Folie versiegelt. Ist das Medikamentenmagazin als Ringmagazin mit mehreren kreisförmig darin angeordneten Medikamentenkammern gestaltet, so befinden sich Steuer- und Entnahmeöffnung jeder Kammer beabstandet zueinander auf einer Seite des Magazins, während die Einfüllöffnung auf einer gegenüberliegenden Seite des Magazins angeordnet ist. Die EP 1 844 806 A1 offenbart des Weiteren in einer speziellen Ausführungsform einen Pulverinhalator mit einem Doppel-Medikamentenmagazin, in dem zwei Ringmagazine mit der Seite ihrer jeweiligen Einfüllöffnungen aneinander anliegen und aneinander festgemacht sind. Dabei sind sie so gegeneinander verschoben, dass die einzelnen Medikamentenkammern nicht genau einander gegenüber liegen sondern radial versetzt sind. Zwei Luftkanäle, welche jeweils zu einem oberen und unteren Teil des Magazins gehören, münden in dasselbe Mundstück, wobei Mundstück und Luftkanäle einstückig aufgebaut sind. Aufgrund der versetzten Anordnung der Medikamentenkammern wird jeweils nur aus einer Kammer Pulver entnommen. Optional kann der nicht im Einsatz stehende Luftkanal für eine zusätzliche Luftzufuhr verwendet werden.

Des Weiteren beschreibt die WO99/36116 ein Gerät zur Inhalation von pulverförmigen Medikamenten, wobei sich das pulverförmige Medikament in vorbestimmten Mengen in Kavitäten in einem ersten Gehäuseteil befindet. Dieses erste Gehäuseteil ist derart gegenüber einem fest angebundenen zweiten Gehäuseteil beweglich (beispielsweise drehbar), dass es eine erste Position gibt, in der die Kavitäten mit dem Medikament geschlossen sind und eine zweite, in der eine Kavität in Verbindung mit einem Luftweg gebracht wird und das Medikament inhaliert werden kann. In einer Ausführungsform ist das Gerät in einer Flüssigkeits-dichten oder luftdichten Verpackung eingeschlossen, die verhindert, dass das Medikament feucht wird.

Es ist Aufgabe der Erfindung einen Inhalator der eingangs genannten Art zu schaffen, der bei einem einfachen Aufbau leicht und zuverlässig handhabbar ist.

Erfindungsgemäß wird die Aufgabe durch einen Inhalator gemäß Anspruch 1 gelöst. Der erfindungsgemäße Inhalator weist ein Mundstück und ein Magazin auf, das ein Gehäuse mit mehreren Kavitäten zur Aufnahme des Arzneimittels umfasst, wobei das geschlossene Gehäuse einen Deckel umfasst, der mindestens einen Träger mit den nicht gesiegelten Kavitäten abdeckt, wobei der Träger relativ zu dem Deckel verlagerbar ist, um eine Kavität in Strömungsverbindung mit dem Mundstück zu bringen.

Zum Gebrauch des Inhalators, der insbesondere als so genannter Mehrdosispulverinhalator ausgebildet ist, ist es nicht erforderlich, ein Siegel von den Kavitäten zu entfernen oder diese anzustechen. Vielmehr sind die Kavitäten mit dem Arzneimittel lediglich durch den Deckel des Gehäuses abgedeckt. Der Erfindung liegt die Erkenntnis zugrunde, dass es nicht zwingend erforderlich ist, das Arzneimittel in den Kavitäten durch das Siegeln mit speziellen Folien vor Feuchtigkeit zu schützen, wenn das Arzneimittel regelmäßig einzunehmen ist und nur so viele Dosen an Arzneimittel durch das Magazin zur Verfügung gestellt werden, wie über eine Lebensdauer des Arzneimittels bei bestimmungsgemäßer Lagerung üblicherweise verbraucht werden.

Inhalatoren sind unter den Markennamen HandiHaler^{®}, Spinhaler^{®}, Rotahaler^{®}, Aerolizer^{®}, Flowcaps^{®}, Turbospin^{®}, AIR DPI^{®}, Orbital^{®}, Directhaler^{®} bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler^{®}, Rotahaler^{®}, Aerolizer^{®}, Inhalator^{®}, HandiHaler^{®}, Diskhaler^{®}, Diskus^{®}, Accuhaler^{®}, Aerohaler^{®}, Eclipse^{®}, Turbohaler^{®}, Turbuhaler^{®}, Easyhaler^{®}, Novolizer^{®}, Clickhaler^{®}, Pulvinal^{®}, Novolizer^{®}, SkyeHaler^{®}, Xcelovair^{®}, Pulvina^{®}, Taifun^{®}, MAGhaler^{®}, Twisthaler^{®} und der Jethaler^{®} bekannt.

Besonders bevorzugt sind in diesem Zusammenhang Arzneimittel, die ausgewählt sind aus der Gruppe bestehend aus Anticholinergika, Betamimetika, Steroiden, Phosphodiesterase IV-inhibitoren, LTD4-Antagonisten und EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen, z.B. Betamimetika plus Anticholinergika oder Betamimetica plus Antiallergika. Im Fall von Kombinationen weist wenigstens einer der Wirkstoffe chemisch gebundenes Wasser auf. Bevorzugt werden anticholinergikahaltige Wirkstoffe eingesetzt, als Monopräparate oder in Form von Kombinationspräparaten.

Im Einzelnen seien als Beispiele für die wirksamen Bestandteile oder deren Salze genannt:
Zur Anwendung gelangende Anticholinergika sind bevorzugt ausgewählt aus der Gruppe bestehend aus Tiotropiumbromid, Oxitropiumbromid, Flutropiumbromid, Ipratropiumbromid, Glycopyrroniumsalze, Trospiumchlorid, Tolterodin, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxyfluoren-9-carbonsäuretropenolester -Methobromid, 9-Fluorfluoren-9-carbonsäuretropenolester -Methobromid, 9-Hydroxyfluoren-9-carbonsäurescopinester -Methobromid, 9-Fluorfluoren-9-carbonsäurescopinester Methobromid, 9-Methylfluoren-9-carbonsäuretropenolester Methobromid, 9-Methylfluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinester-Methobromid,
   9-Methyl-fluoren-9-carbonsäurecyclopropyltropinester-Methobromid,
   9-Methyl-xanthen-9-carbonsäurecyclopropyltropinester-Methobromid,
   9-Hydroxy-fluoren-9-carbonsäurecyclopropyltropinester-Methobromid,
   4,4'-Difluorbenzilsäuremethylestercyclopropyltropinester - Methobromid, 9-Hydroxy-xanthen-9-carbonsäuretropenolester - Methobromid, 9-Hydroxy-xanthen-9-carbonsäurescopinester Methobromid, 9-Methyl-xanthen-9-carbonsäuretropenolester - Methobromid, 9-Methyl-xanthen-9-carbonsäurescopinester - Methobromid, 9-Ethyl-xanthen-9-carbonsäuretropenolester Methobromid, 9-Difluormethyl-xanthen-9-carbonsäuretropenolester -Methobromid und 9-Hydroxymethylxanthen-9-carbonsäurescopinester -Methobromid, gegebenenfalls in Form ihrer Racemate, Enantiomere oder Diastereomere und gegebenenfalls in Form ihrer Salze, Solvate und/oder Hydrate.

Zur Anwendung gelangende Betamimetika sind bevorzugt ausgewählt aus der Gruppe bestehend aus Albuterol, Bambuterol, Bitolterol, Broxaterol, Carbuterol, Clenbuterol, Fenoterol, Formoterol, Hexoprenaline, Ibuterol, Indacterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzolsulfonamid, 5-[2-(5,6-Diethyl-indan-2-ylamino)-1-hydroxy-ethyl]-8-hydroxy-1H-quinolin-2-on, 4-hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol , 1-[3-(4-methoxybenzyl-amino)-4-hydroxyphenyl]-2-[4-(1-benzimidazolyl)-2-methyl-2-butylamino]ethanol , 1-[2H-5-hydroxy-3-oxo-4H-1,4-benzoxazin-8-yl]-2-[3-(4-N,N-dimethylaminophenyl)-2-methyl-2-propylamino]ethanol , 1-[2H-5-hydroxy-3-oxo-4H-1,4-benzoxazin-8-yl]-2-[3-(4-methoxyphenyl)-2-methyl-2-propylamino]ethanol , 1-[2H-5-hydroxy-3-oxo-4H-1,4-benzoxazin-8-yl]-2-[3-(4-n-butyloxyphenyl)-2-methyl-2-propylamino]ethanol , 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol , 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol und 1-(4-ethoxycarbonylamino-3-cyano-5-fluorophenyl)-2-(tert.-butylamino)ethanol, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende Steroide sind bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester und Etiprednol-dichloroacetat (BNP-166), gegebenenfalls in Form ihrer Racemate, Enantiomere oder Diastereomere und gegebenenfalls in Form ihrer Salze und Derivate, ihrer Solvate und/oder Hydrate.

Zur Anwendung gelangende PDE IV-inhibitoren sind bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4aR*,10bS*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Zur Anwendung gelangende LTD4-Antagonisten sind bevorzugt ausgewählt aus der Gruppe bestehend aus Montelukast, 1-(((R)-(3-(2-(6,7-Difluoro-2-quinolinyl)ethenyl)phenyl)-3-(2-(2- hydroxy-2-propyl)phenyl)thio)methylcyclopropanessigsäure, 1-(((1(R)-3(3-(2-(2,3-Dichlorothieno[3,2-b]pyridin-5-yI)-(E)-ethenyl)phenyl)-3-(2-(1-hydroxy-1-methyl-ethyl)phenyl)propyl)thio)methyl)cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Zur Anwendung gelangende EGFR-Kinase-Hemmer sind bevorzugt ausgewählt aus der Gruppe bestehend aus Cetuximab, Trastuzumab, ABX-EGF, Mab ICR-62, 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopropylmethoxy-chinazolin, 4-[(R)-(1-Phenylethyl)amino]-6-({4-[N-(tetrahydropyran-4-yl)-N-methyl-amino]-1-oxo-2-buten-1-yl}amino)-7-cyclopropylmethoxychinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxyethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxomorpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluorphenyl)amino]-6-(trans-4-methansulfonylamino-cyclohexan-1-yloxy)-7-methoxy-chinazolin, 4-[(3-Chlor-4-fluorphenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxychinazolin, 4-[(3-Chlor-4-fluor-phenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluorphenyl)amino]-6-(tetrahydropyran-4-yloxy)-7-ethoxychinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxychinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluorphenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxychinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylaminocyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxychinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Antiallergika: Dinatriumcromoglicat, Nedocromil.

Als Derivate der Mutterkornalkaloide: Dihydroergotamin, Ergotamin.

Für die Inhalation kommen Arzneimittel mit den o.g. Wirkstoffen in Betracht sowie deren Salze, Ester sowie die Kombination dieser Wirkstoffe, Salze und Ester.

Vorzugsweise handelt es sich bei den Kunststoffen, aus denen Einzelteile des Inhalators gefertigt sind, um Polymerisate, thermoplastische Polykondensate, Polyaddukte, abgewandelte Naturstoffe oder Kautschuke bzw. um Gemische dieser Kunststoffe.

Besonders bevorzugt sind hier Polyolefine, Vinylchlorid-Polymerisate, Styrol-Polymerisate, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acrylester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden.

In Ausgestaltung weist der Deckel eine Ausgangsöffnung und eine Eingangsöffnung auf, die zueinander beabstandet im Bereich des Mundstücks angeordnet sind, wobei zumindest die Ausgangsöffnung im Bereich der zu entleerenden napfförmigen Kavität angeordnet ist. Demnach ist der Strömungskanal derart gestaltet, dass zumindest ein Teil der beim Inhalieren erzeugten Entleerungsströmung über das in der Kavität befindliche Medikament hinwegströmt und dieses mitnimmt. Über eine innere Gestaltung der Kavität und/oder der Ausgangs- bzw. Eingangsöffnung und/oder des Mundstücks kann Einfluss auf eine Deagglomeration und Dispersion des Medikamentes genommen werden.

Zweckmäßigerweise weist das an beiden Enden offene Mundstück zwischen der insbesondere langlochförmigen Ausgangsöffnung und der Eingangsöffnung eine Querschnittsreduzierung auf. Ausgehend von einer offenen, dem Nutzer des Inhalators abgewandten Eingangsseite strömt die angesaugte Luft beim Inhalieren sowohl durch die Querschnittsreduzierung als auch durch die Eingangsöffnung in die Kavität und arzneimittelbeladen durch die Ausgangsöffnung zur Ausgangsseite des Mundstücks. Mit der Querschnittsreduzierung wird die Deagglomeration bzw. Dispersion des Arzneimittels im Mundstück aufgrund von Turbulenzen verbessert.

Vorzugsweise ist das Mundstück in einer T-nutförmigen Aufnahme des Deckels, insbesondere lösbar, in einer definierten Lage gehalten. Die T-nutförmige Aufnahme erleichtert die Montage des Mundstücks bzw. dessen Befestigung am Deckel bei der Herstellung des Inhalators. Ist das Mundstück lösbar an dem Deckel befestigt, kann es zur Reinigung von dem Magazin entnommen werden. Es ist auch möglich, bei Bedarf nur das Magazin zu ersetzen und das Mundstück weiter zu verwenden.

Nach einer Weiterbildung umfasst das Gehäuse einen Boden, mit dem der Deckel abgedichtet und unlösbar verbunden ist. Wenn sowohl der Deckel als auch der Boden aus einem Kunststoff bestehen, kann die Verbindung beispielsweise durch Schweißen vorgenommen werden. Selbstverständlich ist der Träger zwischen dem Deckel und dem Boden geschützt aufgenommen.

Bevorzugt weist das Gehäuse einen kreisförmigen Querschnitt auf und lagert den scheibenförmigen Träger verrastend drehbar, wobei dem Gehäuse und dem Träger eine Betätigungseinrichtung zum schrittweisen Verdrehen des Trägers zugeordnet ist, um eine zu entleerende Kavität in den Strömungskanal mit dem Mundstück zu verlagern. Mittels der Betätigungseinrichtung kann der Benutzer den Träger derart verlagern, dass eine mit dem Arzneimittel gefüllte Kavität in den Strömungskanal, also unter die deckelseitige Ausgangsöffnung gelangt und dort eine definierte Position einnimmt. Demnach ist der Inhalator einfach und zuverlässig zu benutzen.

In weiterer Ausgestaltung sind in dem Gehäuse zwei scheibenförmige Träger verrastend drehbar, unmittelbar übereinander liegend drehbar gelagert, wobei der dem Mundstück zugewandte Träger eine Auslassöffnung und eine Einlassöffnung aufweist, die zu der Ausgangsöffnung und der Eingangsöffnung des Deckels korrespondieren. Damit können eine Vielzahl zu verabreichender Dosen eines Arzneimittels in einem kompakt aufgebauten Magazin gelagert werden. Das Arzneimittel aus dem bodenseitigen Träger gelangt durch die Auslassöffnung in dem mundstückseitigen Träger und die Ausgangsöffnung im Deckel in das Mundstück. Gleichzeitig wird die angesaugte Luft durch die Eingangsöffnung des Deckels und die Einlassöffnung des mundstückseitigen Trägers zu der Kavität geleitet, wobei selbstverständlich die beiden Träger mit ihren einander zugewandten Stirnseiten unmittelbar aufeinander liegen, um Strömungs- bzw. Arzneimittelverluste zu vermeiden.

Zur Bereitstellung einer einfach aufgebauten Lagerung weist der dem Mundstück zugewandte Träger einen umfangsseitigen Bund mit einem gegenüber den Trägern vergrößerten Außendurchmesser auf, in dem zum einen der bodenseitige Träger mit seiner die Kavitäten aufweisenden Stirnseite gelagert ist und der sich zum anderen im Gehäuse abstützt. Selbstverständlich kann zur exakten Führung auch eine zusätzliche Achse das gesamte Gehäuse des Magazins mit seinen Trägern durchragen.

Vorteilhafterweise umfasst die Betätigungseinrichtung ein im Boden auf einer Kreisbahn verschiebbar gelagertes Betätigungselement, das über einen Federarm in äquidistante Ausnehmungen des bodenseitigen Trägers eingreift, und die beiden Träger weisen Mitnehmerzapfen auf, die in gegenseitige Anlage kommen, wenn der bodenseitige Träger aus einer Ausgangslage um eine Umdrehung verdreht ist. Damit ist eine einfach aufgebaute Betätigungseinrichtung zur zuverlässigen Verdrehung der beiden Träger in dem Gehäuse realisiert.

Zur Verwirklichung einer genauen Positionierung ist zweckmäßigerweise zum schrittweisen Verdrehen der beiden Träger jedem Träger mindestens ein mit dem Umfang des topfförmigen Deckels zusammenwirkender Federarm zugeordnet. Insbesondere sind zwei diametral gegenüberliegende Federarme an jedem Träger vorgesehen, wobei die Träger beispielsweise einstückig mit den Federarmen im Spritzgußverfahren gefertigt sein können.

Zwecks weiterer Verbesserung der Handhabung des Inhalators und zur Sicherstellung der Wirksamkeit des Arzneimittels sind innerhalb des Gehäuses ein Trockenmittel und/oder eine, insbesondere digitale, Feuchteanzeige angeordnet. Selbstverständlich kann das Trockenmittel, das keine negativen Auswirkungen auf das im Magazin des Inhalators eingelagerte Arzneimittel aufweist, in einer speziellen Kammer untergebracht sein. Die Feuchteanzeige stellt eine für den Benutzer optisch wahrnehmbare Information bezüglich der Luftfeuchtigkeit innerhalb des Gehäuses dar, anhand der sich beispielsweise die Brauchbarkeit und/oder Unbrauchbarkeit des Arzneimittels bestimmen lässt. Eine digitale Feuchteanzeige kann wechselnde Farben, insbesondere grün und rot, oder einen schriftlichen Warnhinweis umfassen.

Um eine möglichst gleichmäßige Verteilung der pharmazeutischen Pulverformulierung zu erhalten, ist das Mundstück mit einer Dispergiereinheit ausgestattet.

Zweckmäßigerweise ist jede Kavität zur Aufnahme von ca. 50mg des Arzneimittels ausgelegt und das Magazin umfasst vorzugsweise 30 Kavitäten. Die Kavitäten sind unter Einbeziehung einer Leerposition, die bei dem deckelseitigen Träger die Auslassöffnung und die Einlassöffnung aufweist, äquidistant über den Umfang der Träger verteilt, wobei jeder Träger 15 Kavitäten aufweist. Prinzipiell ist auch jede andere Anzahl von Kavitäten möglich, beispielsweise vier, sechs, acht, zehn, zwölf, 16, 20, 24 oder 32.

Zur Kontrolle des Inhalts verschiedener Kavitäten im Magazin des Inhalators ist das Gehäuse zumindest abschnittsweise transparent ausgebildet, um durch diesen transparenten Abschnitt hindurch optisch überprüfen zu können, ob eine oder mehrere Kavitäten bereits leer oder noch voll sind. Beispielsweise ist der transparente Abschnitt derart angeordnet, dass stets jene Kavität einsehbar ist, auf die als nächstes über das Mundstück zugegriffen wird, die sich also in Drehrichtung der Träger gesehen vor einer Entnahme- bzw. Inhalationsposition befindet.

Um das Arzneimittel in den Kavitäten der Träger des Magazins relativ langfristig vor Feuchtigkeit zu schützen, ist eine entfernbare, feuchtigkeitsundurchlässige Umverpackung für den Inhalator und/oder das Magazin vorgesehen.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispiels unter Bezugnahme auf die zugehörigen Zeichnungen näher erläutert. Es zeigt:

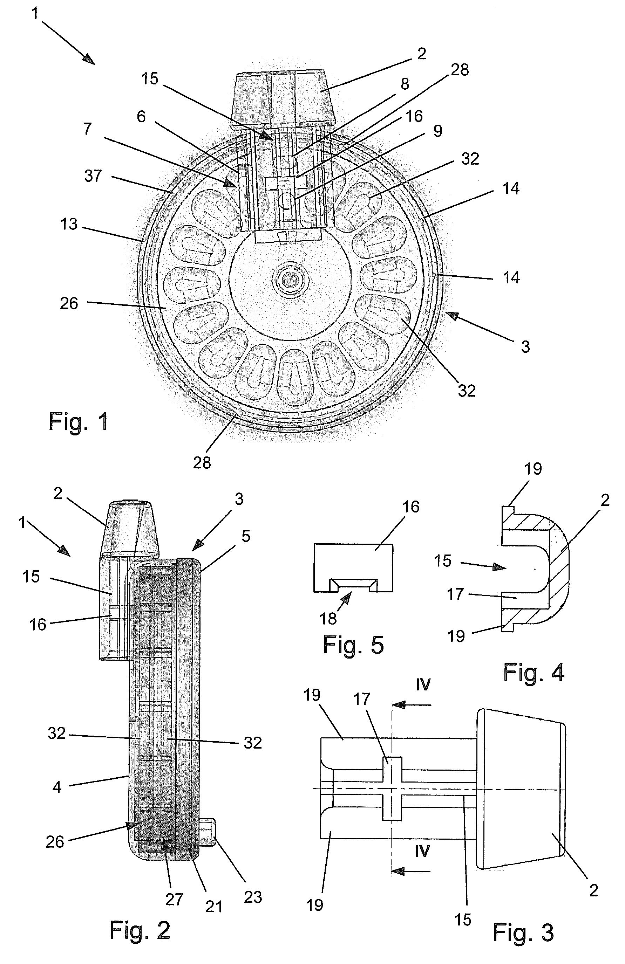

- Fig.1: eine Draufsicht auf einen erfindungsgemäßen Inhalator,
- Fig.2: eine Seitenansicht des Inhalators nach Fig. 1,
- Fig.3: eine Draufsicht auf ein Mundstück des Inhalators nach Fig. 1,
- Fig.4: eine Schnittdarstellung des Mundstücks gemäß der Linie IV-IV nach Fig. 3,
- Fig.5: eine Draufsicht auf eine Drossel für das Mundstück nach Fig. 3,

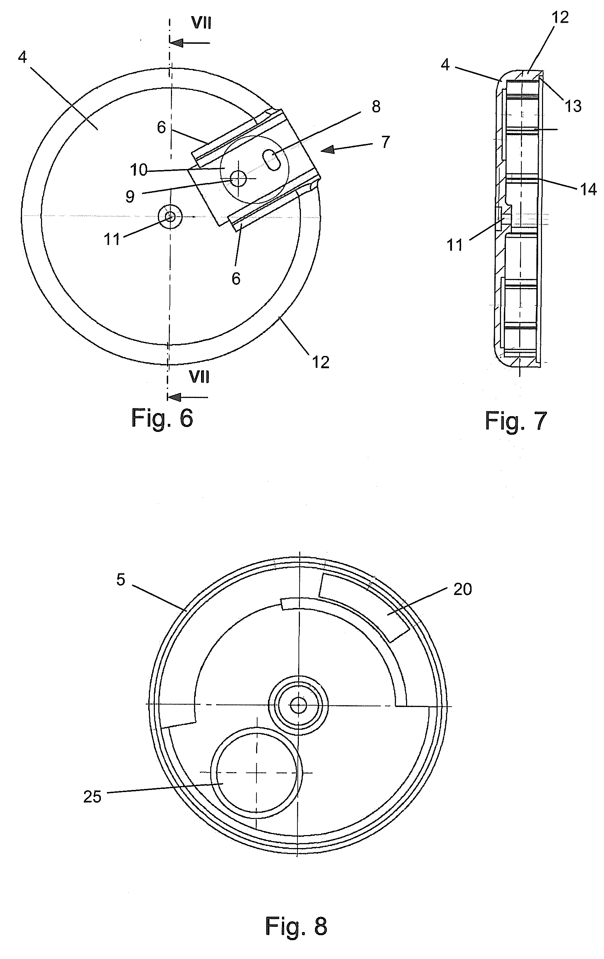

- Fig.6: eine Draufsicht auf einen Deckel des Inhalators nach Fig. 1,
- Fig.7: eine Schnittdarstellung des Deckels gemäß der Linie VII-VII nach Fig. 6,
- Fig.8: eine Draufsicht auf einen Boden des Inhalators nach Fig. 1,

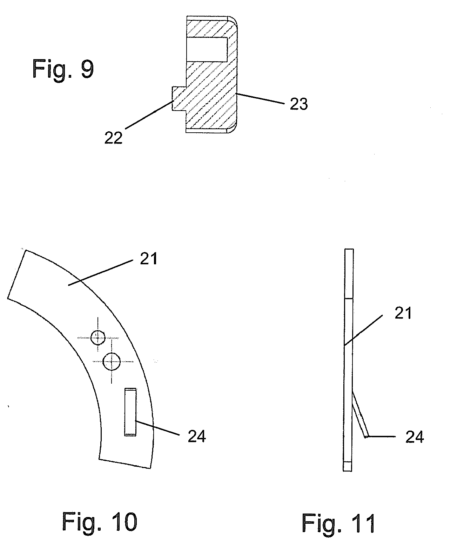

- Fig.9: eine Schnittdarstellung eines Betätigers des Inhalators nach Fig. 1,
- Fig.10: eine Draufsicht auf ein Betätigungselement des Inhalators nach Fig. 1,
- Fig.11: eine Seitenansicht des Betätigungselements nach Fig. 10,

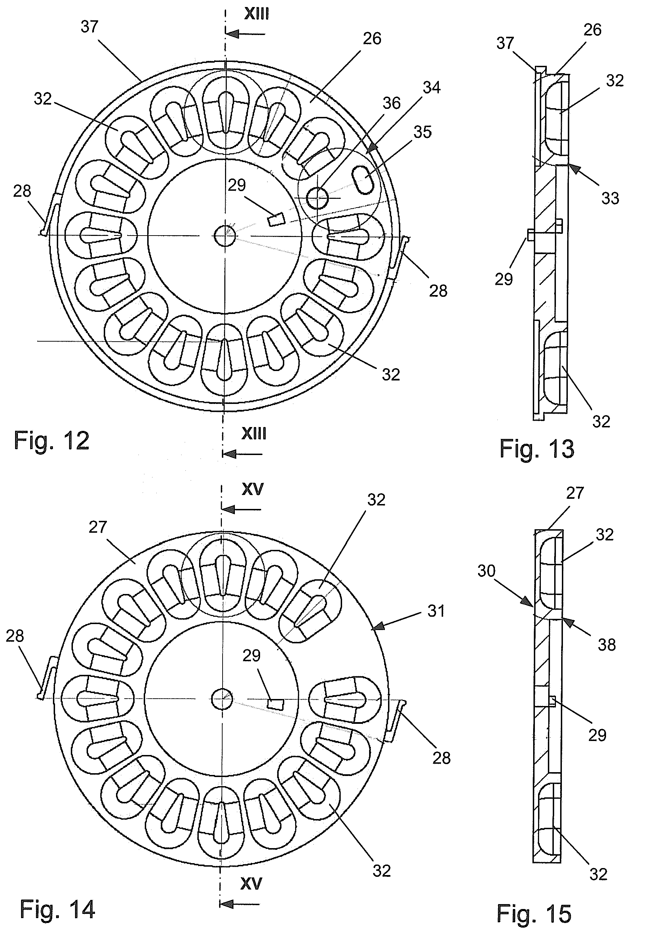

- Fig.12: eine Draufsicht auf einen Träger des Inhalators nach Fig. 1,
- Fig.13: eine Schnittdarstellung des Trägers gemäß der Linie XIII-XIII nach Fig. 12,
- Fig.14: eine Draufsicht auf einen weiteren Träger des Inhalators nach Fig. 1 und
- Fig.15: eine Schnittdarstellung des Trägers gemäß der Linie XV-XV nach Fig. 14

Der Inhalator 1 ist als so genannter Mehrdosispulverinhalator zur Verabreichung von einem pulverförmigen Arzneimittel in Form einer inhalationsfähigen Substanz, Substanzformulierung oder -mischung ausgebildet und umfasst ein Mundstück 2, das mit einem Gehäuse 3 eines Magazins für die Aufnahme mehrerer Dosen des Arzneimittels verbunden ist. Das Gehäuse 3 besteht im Wesentlichen aus einem Deckel 4 und einem fest damit verbundenen Boden 5, wobei der Deckel 4 zwei Schenkel 6 zur Bildung einer T-nutförmigen Aufnahme 7 für das Mundstück 2 aufweist. Zwischen den beiden Schenkeln 6 sind eine langlochförmige Ausgangsöffnung 8 und eine Eingangsöffnung 9 für einen Luftstrom beabstandet zueinander in den Deckel 4 eingelassen. Im Bereich der Ausgangs- 8 und Eingangsöffnung 9 ist eine erhabene Dichtfläche 10, auf der das Mundstück 2 bereichsweise aufliegt, auf dem Deckel 4 ausgebildet. Im Zentrum des Deckels 4 befindet sich eine Bohrung 11 zur Aufnahme einer das Gehäuse 3 durchragenden Achse. Der Deckel 3 ist im Wesentlichen topfförmig gestaltet, wobei an der umfangsseitigen Wandung 12 ein Ansatz 13 zur Zentrierung des Bodens 5 und äquidistant verteilte Einbuchtungen 14 ausgeformt sind.

Das Mundstück 2 weist einen sich durch seine Längsachse erstreckenden Kanal 15 auf, in den eine den Querschnitt des Kanals 15 reduzierenden Drossel 16 eingesetzt. Senkrecht zur Längsachse des Mundstücks 2 ist eine Aussparung 17 in das Mundstück 2 eingearbeitet, in die die Drossel 16 derart eingesetzt ist, dass sie sich in der am Deckel 4 montierten Position des Mundstücks 2 zwischen der Ausgangs- 8 und der Eingangsöffnung 9 des Deckels 4 befindet und durch eine Vertiefung 18 in der Drossel 16 einen Strömungskanal bildet. Der zu befestigende Teil des Mundstücks 2 weist beidseitig Schenkel 19 zum Einschieben in die T-nutförmigen Aufnahme 7 des Deckels 4 auf.

Der Boden 5 ist mit einer kreissegmentförmigen Öffnung 20 versehen, die ein mit einem ebenfalls kreissegmentförmigen Betätigungselement 21 über Bolzen 22 verbundener Betätiger 23 durchragt, wobei das Betätigungselement 21 einen in das Gehäuse 3 ragenden Federarm 24 aufweist. Im Weiteren ist am Boden 5 eine Kammer 25 für ein Trockenmittel ausgebildet.

In dem Gehäuse 3 mit einem kreisförmigen Querschnitt sind zwei unmittelbar übereinander angeordnete kreisförmige Träger 26, 27 drehbar gelagert, an denen jeweils zwei diametral gegenüberliegende Federarme 28 angeordnet sind, die zum schrittweisen Positionieren der Träger 26, 27 mit den umfangsseitigen Einbuchtungen 14 des topfförmigen Deckels 4 zusammenwirken. Im Weiteren weist jeder Träger 26, 27 einen Mitnehmerzapfen 29 auf, der mit dem Mitnehmerzapfen 29 des jeweils anderen Trägers 26, 27 zusammenwirken kann.

Der bodenseitige Träger 27 weist auf seiner dem Boden 5 zugewandten Stirnseite 30 äquidistant verteilte Ausnehmungen auf, in die der Federarm 24 des Betätigungselementes 21 eingreift. Im Weiteren sind unter Einbeziehung einer Leerposition 31 gleichmäßig über den Umfang des Trägers 27 verteilte napfförmige Kavitäten 32 zur Aufnahme des Arzneimittels angeordnet.

In dem deckelseitigen Träger 26 sind auf der dem Deckel 4 zugewandten Stirnseite 33 ebenfalls die gleichmäßig über den Umfang des Trägers 26 verteilten Kavitäten 32 ausgeformt, wobei in einer Position 34 statt einer Kavität 32 eine Auslassöffnung 35 und eine Einlassöffnung 36 vorgesehen sind, die zu der Ausgangsöffnung 8 und der Eingangsöffnung 9 des Deckels 4 korrespondieren, wobei die Auslassöffnung 35 sowie die Ausgangsöffnung 8 jeweils langlochförmig und die Einlassöffnung 36 sowie die Eingangsöffnung 9 jeweils zylindrisch geformt sind. Des Weiteren weist der dem Mundstück 2 zugewandte Träger 26 einen umfangsseitigen Bund 37 mit einem gegenüber den Trägern 26, 27 vergrößerten Außendurchmesser auf, in dem zum einen der bodenseitige Träger 27 mit seiner die Kavitäten 32 aufweisenden Stirnseite 38 gelagert ist und der sich zum anderen im Deckel 4 des Gehäuses 3 abstützt.

In einem Ausgangszustand des Inhalators 1, in dem alle Kavitäten 32 mit dem Arzneimittel gefüllt sind, befinden sich die Auslassöffnung 35 und die Einlassöffnung 36 des deckelseitigen Trägers 26 in einer zu der Ausgangsöffnung 8 und der Eingangsöffnung 9 des Deckels 4 fluchtenden Position und die Leerposition 31 des bodenseitigen Trägers 27 ist unterhalb der Position 34 des deckelseitigen Trägers 26 mit der Ausgangsöffnung 8 und der Eingangsöffnung 9 ausgerichtet. Da der Deckel 4 unmittelbar auf der zugeordneten Stirnseite 33 des deckelseitigen Trägers 26 und der deckelseitige Träger 26 unmittelbar stirnseitig auf dem bodenseitigen Träger 27 aufliegt, kann kein Arzneimittel aus den Kavitäten 32 entweichen.

Zur Benutzung des Inhalators wird das Betätigungselement 21 mit dem Betätiger 23 verschoben, wobei der Federarm 24 des Betätigungselementes 21 in eine der Ausnehmungen des bodenseitigen Träger 27 eingreift und diesen soweit verdreht, dass die Federarme 28 in den entsprechenden Einbuchtungen 14 des Deckels 4 verrasten, um die der Drehstellung zugeordnete Kavität 32 unter der Auslassöffnung 35 und der Einlassöffnung 36 des deckelseitigen Trägers 26 zu positionieren. Der Benutzer saugt durch den Kanal 15 des Mundstücks 2 Luft, die sowohl durch die Drossel 16 als auch die Eingangsöffnung 8 des Deckels 4 und die Einlassöffnung 35 des mundstückseitigen Trägers 26 zu der zu leerenden Kavität 32 geleitet wird, um das Arzneimittel durch die Auslassöffnung 35 des deckelseitigen Trägers 26, die Ausgangsöffnung 8 des Deckels 4 und den Kanal 15 des Mundstücks 2 zu dem Benutzer zu transportieren. In dieser Stellung, in der die entleerte Kavität 32 unterhalb der Auslassöffnung 35 und der Einlassöffnung 36 des deckelseitigen Trägers 26 ausgerichtet ist, kann der Inhalator 1 transportiert werden. Durch eine Hinund Herbewegung des Betätigers 24 wird der bodenseitige Träger 27 verdreht, um die nächste Kavität 32 unter der Auslassöffnung 35 und der Einlassöffnung 36 des deckelseitigen Trägers 26 zu positionieren. Sind alle Kavitäten 32 des bodenseitigen Trägers 27 geleert, kommen die beiden Mitnehmerzapfen 29 in gegenseitigen Eingriff und mit einer Drehbewegung des bodenseitigen Trägers 27 geht eine Drehbewegung des deckelseitigen Trägers 26 einher, um dessen Kavitäten 32 zur Entleerung unterhalb der Ausgangsöffnung 8 und der Eingangsöffnung 9 des Deckels 4 anzuordnen, wobei bei einer Verdrehung der beiden Träger 26, 27 zur genauen Positionierung der Näpfe 32 sämtliche Federarme 28 wirksam sind.

**Bezugszeichenliste**

| | | | |
|---|---|---|---|
| 1. | Inhalator | 28. | Federarm von 26, 27 |
| 2. | Mundstück | 29. | Mitnehmerzapfen |
| 3. | Gehäuse | 30. | Stirnseite von 27 |
| 4. | Deckel | 31. | Leerposition |
| 5. | Boden | 32. | Kavität |
| 6. | Schenkel von 7 | 33. | Stirnseite von 26 |
| 7. | Aufnahme | 34. | Position |
| 8. | Ausgangsöffnung | 35. | Auslassöffnung |
| 9. | Eingangsöffnung | 36. | Einlassöffnung |
| 10. | Dichtfläche | 37. | Bund |
| 11. | Bohrung | 38. | Stirnseite von 27 |
| 12. | Wandung von 4 | | |
| 13. | Ansatz | | |
| 14. | Einbuchtung | | |
| 15. | Kanal | | |
| 16. | Drossel | | |
| 17. | Aussparung | | |
| 18. | Vertiefung von 16 | | |
| 19. | Schenkel von 2 | | |
| 20. | Öffnung | | |
| 21. | Betätigungselement | | |
| 22. | Bolzen | | |
| 23. | Betätiger | | |
| 24. | Federarm | | |
| 25. | Kammer | | |
| 26. | Träger | | |
| 27. | Träger | | |

## Patentansprüche

1. Pulverinhalator, zur Verabreichung eines Arzneimittels in Form inhalationsfähiger Substanzen, Substanzformulierungen oder -mischungen mit einem Mundstück (2) und einem Magazin, das ein Gehäuse (3) mit mehreren Kavitäten (32) zur Aufnahme des Arzneimittels umfasst,
wobei das Mundstück (2) mit jeweils einer der Kavitäten (32) in Strömungsverbindung steht,
wobei das geschlossene Gehäuse (3) einen Deckel (4) umfasst, der mindestens einen Träger (26, 27) mit den nicht gesiegelten Kavitäten (32) abdeckt, wobei der Träger (26, 27) relativ zu dem Deckel (4) verlagerbar ist, um eine Kavität (32) in Strömungsverbindung mit dem Mundstück (2) zu bringen, und wobei zwei scheibenförmige Träger (26, 27) unmittelbar übereinander liegend angeordnet sind,
**dadurch gekennzeichnet,**
**dass** die zwei scheibenförmige Träger (26, 27) verrastend drehbar in dem Gehäuse (3) gelagert sind, wobei ein Träger (26) dem Mundstück (2) zugewandt und der andere Träger (27) bodenseitig im Gehäuse angeordnet ist, und wobei der dem Mundstück (2) zugewandte Träger (26) eine Auslassöffnung (35) und eine Einlassöffnung (36) aufweist, die zu einer Ausgangsöffnung (8) und einer Eingangsöffnung (9) im Deckel (4) korrespondieren, wobei Arzneimittel aus dem bodenseitigen Träger (27) durch die Auslassöffnung (35) in dem mundstückseitigen Träger (26) und die Ausgangsöffnung (8) im Deckel (4) in das Mundstück (2) gelangen kann und gleichzeitig angesaugte Luft durch die Eingangsöffnung (9) des Deckels (4) und die Einlassöffnung (36) des mundstückseitigen Trägers (26) zu der Kavität (32) geleitet werden kann.

2. Inhalator nach Anspruch 1, **dadurch gekennzeichnet, dass** der Deckel (4) eine Ausgangsöffnung (8) und eine Eingangsöffnung (9) aufweist, die zueinander beabstandet im Bereich des Mundstücks (2) angeordnet sind, wobei zumindest die Ausgangsöffnung (8) im Bereich der zu entleerenden napfförmigen Kavität (32) angeordnet ist.

3. Inhalator nach Anspruch 1 oder 2, **dadurch gekennzeichnet, dass** das an beiden Enden offene Mundstück (2) zwischen der insbesondere langlochförmigen Ausgangsöffnung (8) und der Eingangsöffnung (9) eine Querschnittsreduzierung aufweist.

4. Inhalator nach einem der Ansprüche 1 bis 3, **dadurch gekennzeichnet, dass** das Mundstück (2) in einer T-nutförmigen Aufnahme (7) des Deckels (4), insbesondere lösbar, in einer definierten Lage gehalten ist.

5. Inhalator nach einem der Ansprüche 1 bis 4, **dadurch gekennzeichnet, dass** das Gehäuse (3) einen Boden (5) umfasst, mit dem der Deckel (4) abgedichtet und unlösbar verbunden ist.

6. Inhalator nach einem der Ansprüche 1 bis 5, **dadurch gekennzeichnet, dass** das Gehäuse (3) einen kreisförmigen Querschnitt aufweist und den scheibenförmigen Träger (26, 27) verrastend drehbar lagert, wobei dem Gehäuse (3) und dem Träger (26, 27) eine Betätigungseinrichtung zum schrittweisen Verdrehen des Trägers (26, 27) zugeordnet ist, um eine zu entleerende Kavität (32) in den Strömungskanal mit dem Mundstück (2) zu verlagern.

7. Inhalator nach einem der Ansprüche 1 bis 6, **dadurch gekennzeichnet, dass** der dem Mundstück (2) zugewandte Träger (26) einen umfangsseitigen Bund (37) mit einem gegenüber den Trägern (26, 27) vergrößerten Außendurchmesser aufweist, in dem zum einen der bodenseitige Träger (27) mit seiner die Kavitäten (32) aufweisenden Stirnseite (38) gelagert ist und der sich zum anderen im Gehäuse (3) abstützt.

8. Inhalator nach einem der Ansprüche 1 bis 7, **dadurch gekennzeichnet, dass** die Betätigungseinrichtung ein im Boden (5) auf einer Kreisbahn verschiebbar gelagertes Betätigungselement (21) umfasst, das über einen Federarm (24) in äquidistante Ausnehmungen des bodenseitigen Trägers (27) eingreift, und die beiden Träger (26, 27) Mitnehmerzapfen (29) aufweisen, die in gegenseitige Anlage kommen, wenn der bodenseitige Träger (27) aus einer Ausgangslage um eine Umdrehung verdreht ist.

9. Inhalator nach einem der Ansprüche 1 bis 8, **dadurch gekennzeichnet, dass** zum schrittweisen Verdrehen der beiden Träger (26, 27) jedem Träger (26, 27) mindestens ein mit dem Umfang des topfförmigen Deckels (4) zusammenwirkender Federarm (28) zugeordnet ist.

10. Inhalator nach einem der Ansprüche 1 bis 9, **dadurch gekennzeichnet, dass** innerhalb des Gehäuses (3) ein Trockenmittel und/oder eine, insbesondere digitale, Feuchteanzeige angeordnet sind.

11. Inhalator nach einem der Ansprüche 1 bis 10, **dadurch gekennzeichnet, dass** das Mundstück (2) mit einer Dispergiereinheit ausgestattet ist.

12. Inhalator nach einem der Ansprüche 1 bis 11, **dadurch gekennzeichnet, dass** jede Kavität (32) zur Aufnahme von ca. 50mg einer pharmazeutischen Pulverformulierung ausgelegt ist und das Magazin vorzugsweise 30 Kavitäten (32) umfasst.

13. Inhalator nach einem der Ansprüche 1 bis 12, **dadurch gekennzeichnet, dass** das Gehäuse (3) zumindest abschnittsweise transparent ausgebildet ist.

14. Inhalator nach einem der Ansprüche 1 bis 13, **gekennzeichnet durch** eine entfernbare, feuchtigkeitsundurchlässige Umverpackung und/oder eine Umverpackung für das Magazin.

15. Inhalator nach einem der Ansprüche 1 bis 13, **dadurch gekennzeichnet, dass** durch eine Hin- und Herbewegung eines Betätigers (24) der bodenseitige Träger (27) verdreht wird, um die nächste Kavität (32) unter der Auslassöffnung (35) und der Einlassöffnung (36) des deckelseitigen Trägers (26) zu positionieren und dass, wenn alle Kavitäten (32) des bodenseitigen Trägers (27) geleert sind, zwei Mitnehmerzapfen (29) in gegenseitigen Eingriff kommen und dann mit einer Drehbewegung des bodenseitigen Trägers (27) eine Drehbewegung des deckelseitigen Trägers (26) einhergeht, um dessen Kavitäten (32) zur Entleerung unterhalb der Ausgangsöffnung (8) und der Eingangsöffnung (9) der Deckels (4) anzuordnen.

## Claims

1. Powder inhaler for administering a medicament in the form of inhalable substances, substance formulations or mixtures, with a mouthpiece (2) and a magazine that comprises a housing (3) having a plurality of cavities (32) for holding the medicament, the mouthpiece (2) being in flow connection with one of the cavities (32), the closed housing (3) having a cover (4) that covers at least one carrier (26, 27) comprising the unsealed cavities (32), the carrier (26, 27) being movable relative to the cover (4) for bringing a cavity (32) into flow connection with the mouthpiece (2), and two disc-shaped carriers (26, 27) being arranged directly above one another,
**characterised in that**
the two disc-shaped carriers (26, 27) are rotatably mounted in locking manner in the housing (3), one carrier (26) facing the mouthpiece (2) and the other carrier (27) on the base side being arranged in the housing, and wherein the carrier (26) facing the mouthpiece (2) comprises an outlet opening (35) and an inlet opening (36) which correspond to an exit opening (8) and an entry opening (9) in the cover (4), whereby medicament from the carrier (27) on the base side is able to pass through the outlet opening (35) in the carrier (26) on the mouthpiece side and the exit opening (8) in the cover (4) into the mouthpiece (2) and at the same time air aspirated through the entry opening (9) of the cover (4) and the inlet opening (36) of the carrier (26) on the mouthpiece side can be conveyed to the cavity (32).

2. Inhaler according to claim 1, **characterised in that** the cover (4) comprises an exit opening (8) and an entry opening (9), which are arranged at a spacing from each other in the region of the mouthpiece (2), while at least the exit opening (8) is arranged in the region of the cup-shaped cavity (32) that is to be emptied.

3. Inhaler according to claim 1 or 2, **characterised in that** the mouthpiece (2) which is open at both ends has a cross-sectional constriction between the in particular slot-shaped exit opening (8) and the entry opening (9).

4. Inhaler according to one of claims 1 to 3, **characterised in that** the mouthpiece (2) is held, more particularly releasably, in a defined position in a receptacle (7) in the form of a T-shaped groove in the cover (4).

5. Inhaler according to one of claims 1 to 4, **characterised in that** the housing (3) comprises a base (5) to which the **cover (4) is** non-removably attached in sealed manner.

6. Inhaler according to one of claims 1 to 5, **characterised in that** the housing (3) has a circular cross-section and provides a latching rotatable mounting for the disc-shaped carrier (26, 27), while an actuating device for stepwise rotation of the carrier (26, 27) is associated with the housing (3) and the carrier (26, 27) so as to move a cavity (32) that is to be emptied into the flow channel with the mouthpiece (2).

7. Inhaler according to one of claims 1 to 6, **characterised in that** the carrier (26) facing the mouthpiece (2) comprises a circumferential flange (37) with an external diameter greater than that of the carriers (26, 27), in which on the one hand the carrier (27) on the base side is mounted with its end face (38) comprising the cavities (32) and on the other hand is supported in the housing (3).

8. Inhaler according to one of claims 1 to 7, **characterised in that** the actuating device comprises an actuating element (21) that is mounted in the base (5) so as to be movable along a circular path, this actuating element engaging via a spring arm (24) in equidistant recesses in the carrier (27) on the base side, and the two carriers (26, 27) have tappets (29) that move into mutual abutment when the carrier (27) on the base side is rotated through one revolution out of a starting position.

9. Inhaler according to one of claims 1 to 8, **characterised in that,** for stepwise rotation of the two carriers (26, 27), at least one spring arm (28) cooperating with the circumference of the pot-shaped cover (4) is associated with each carrier (26, 27).

10. Inhaler according to one of claims 1 to 9, **characterised in that** a desiccant and/or a, more particularly, digital moisture indicator is or are provided inside the housing (3).

11. Inhaler according to one of claims 1 to 10, **characterised in that** the mouthpiece (2) is fitted with a dispersing unit.

12. Inhaler according to one of claims 1 to 11, **characterised in that** each cavity (32) is designed to hold about 50 mg of a pharmaceutical powdered formulation and the magazine preferably comprises 30 cavities (32).

13. Inhaler according to one of claims 1 to 12, **characterised in that** the housing (3) is constructed to be at least partly transparent.

14. Inhaler according to one of claims 1 to 13, **characterised by** a removable moistureproof outer wrapper and/or an outer wrapper for the magazine.

15. Inhaler according to one of claims 1 to 13, **characterised in that** the carrier (27) on the base side is rotated by a back and forth movement of an actuator (24) in order to position the next cavity (32) underneath the outlet opening (35) and the inlet opening (36) of the carrier (26) on the cover side, and **in that**, when all the cavities (32) of the carrier (27) on the base side have been emptied, two tappets (29) move into mutual engagement and then a rotary movement of the carrier (27) on the base side is accompanied by a rotary movement of the carrier (26) on the cover side, in order to arrange its cavities (32) for emptying underneath the exit opening (8) and the entry opening (9) of the cover (4).

## Revendications

1. Inhalateur de poudre, pour l'administration d'un médicament sous forme de substances, de formulations de substances ou de mélanges de substances inhalables, pourvu d'un embout (2) et d'un magasin comprenant un boîtier (3) à plusieurs cavités (32) pour la réception du médicament,
l'embout (2) étant en communication fluidique avec chacune des cavités (32),
le boîtier (3) comprenant un couvercle (4) qui recouvre au moins un support (26, 27) avec les cavités (32) non scellées, ledit support (26, 27) étant déplaçable par rapport au couvercle (4) pour mettre une cavité (32) en communication fluidique avec l'embout (2), et deux supports (26, 27) en forme de disque sont étant directement superposés l'un à l'autre,
**caractérisé en ce que**
les deux supports (26, 27) en forme de disque sont montés dans le boîtier (3) de manière à être rotatifs et enclenchables, un support (26) étant opposé à l'embout (2) et l'autre support (27) étant disposé dans le fond du boîtier, et le support (26) opposé à l'embout (2) présentant une ouverture d'évacuation (35) et une ouverture d'admission (36) correspondant respectivement à une ouverture de sortie (8) et à une ouverture d'entrée (9) du couvercle (4), un médicament pouvant parvenir par l'ouverture d'évacuation (35) dans le support (26) côté embout depuis le support (27) côté fond et l'ouverture de sortie (8) du couvercle (4) pouvant pénétrer dans l'embout (2), et l'air simultanément aspiré pouvant être conduit vers la cavité (32) par l'ouverture d'entrée (9) du couvercle (4) et l'ouverture d'admission (36) du support (26) côté embout.

2. Inhalateur selon la revendication 1, **caractérisé en ce que** le couvercle (4) présente une ouverture de sortie (8) et une ouverture d'entrée (9) espacées l'une par rapport à l'autre au niveau de l'embout (2), au moins l'ouverture de sortie (8) étant située dans la zone cavité (32) en forme de godet à vider.

3. Inhalateur selon la revendication 1 ou 2, **caractérisé en ce que** l'embout (2) ouvert aux deux extrémités présente une réduction de section transversale entre l'ouverture de sortie (8) notamment en forme de trou oblong et l'ouverture d'entrée (9).

4. Inhalateur selon l'une des revendications 1 à 3, **caractérisé en ce que** l'embout (2) est maintenu de manière notamment amovible en position définie dans une réception (7) en forme de rainure en T du couvercle (4).

5. Inhalateur selon l'une des revendications 1 à 4, **caractérisé en ce que** le boîtier (3) comporte un fond (5) auquel le couvercle (4) est raccordé de manière étanche et inamovible.

6. Inhalateur selon l'une des revendications 1 à 5, **caractérisé en ce que** le boîtier (3) présente une section transversale circulaire et reçoit le support (26, 27) en forme de disque de manière rotative et enclenchable, un dispositif d'actionnement étant associé au boîtier (3) et au support (26, 27) pour la rotation graduelle du support (26, 27), afin de décaler une cavité (32) à vider vers le canal d'écoulement avec l'embout (2).

7. Inhalateur selon l'une des revendications 1 à 6, **caractérisé en ce que** le support (26) opposé à l'embout (2) présente un épaulement (37) périphérique avec un diamètre extérieur agrandi par rapport au support (26, 27), où le support (27) côté fond est logé avec sa face frontale (38) présentant les cavités (32), d'une part, et qui s'appuie sur le boîtier (3), d'autre part.

8. Inhalateur selon l'une des revendications 1 à 7, **caractérisé en ce que** le dispositif d'actionnement comporte un élément d'actionnement (21) monté dans le fond (5) de manière à coulisser sur une trajectoire circulaire, lequel s'engage par un bras de ressort (24) dans des évidements équidistants du support (27) côté fond, et **en ce que** les deux supports (26, 27) présentent des ergots d'entraînement (29) qui viennent en butée réciproque quand le support (27) côté fond est tourné d'un tour à partir d'une position initiale.

9. Inhalateur selon l'une des revendications 1 à 8, **caractérisé en ce que** pour la rotation graduelle des deux supports (26, 27), au moins un bras de ressort (28) coopérant avec la périphérie du couvercle (4) en forme de godet est associé à chaque support (26, 27).

10. Inhalateur selon l'une des revendications 1 à 9, **caractérisé en ce qu'**un moyen de séchage et/ou un affichage d'humidité notamment numérique sont disposés à l'intérieur du boîtier (3).

11. Inhalateur selon l'une des revendications 1 à 10, **caractérisé en ce que** l'embout (2) est pourvu d'une unité de dispersion.

12. Inhalateur selon l'une des revendications 1 à 11, **caractérisé en ce que** chaque cavité (32) est conçue pour la réception d'environ 50 mg d'une formulation de poudre pharmaceutique, et **en ce que** le magasin comprend préférentiellement 30 cavités (32).

13. Inhalateur selon l'une des revendications 1 à 12, **caractérisé en ce que** le boîtier (3) est réalisé au moins partiellement transparent.

14. Inhalateur selon l'une des revendications 1 à 13, **caractérisé par** un suremballage retirable, imperméable à l'humidité et/ou par un suremballage pour le magasin.

15. Inhalateur selon l'une des revendications 1 à 13, **caractérisé en ce que** par mouvement de va-et-vient d'un actionneur (24), le support (27) côté fond est tourné pour positionner la cavité (32) suivante sous l'ouverture d'évacuation (35) et l'ouverture d'admission (36) du support (26) côté couvercle et **en ce que**, lorsque toutes les cavités (32) du support (27) côté fond sont vidées, deux ergots d'entraînement (29) viennent en prise réciproque et une rotation du support (26) côté couvercle est ensuite entraînée par une rotation du support (27) côté fond, pour en disposer les cavités (32) pour vidange sous l'ouverture de sortie (8) et l'ouverture d'entrée (9) du couvercle (4).
